# EUROPÄISCHE PATENTSCHRIFT

(11) **EP 2 047 798 B1**
(45) Veröffentlichungstag und Bekanntmachung des Hinweises auf die Patenterteilung: **17.11.2010**
(21) Anmeldenummer: 07118411.3
(22) Anmeldetag: 12.10.2007
(51) Int. Cl.: A61B 5/151

(54) **Testbandvorrichtung zum Untersuchen einer Körperflüssigkeit**
Test band device for investigating a bodily fluid
Dispositif de bande de test destinés à l'analyse d'un liquide corporel

(43) Veröffentlichungstag der Anmeldung: 15.04.2009
(73) Patentinhaber: F.Hoffmann-La Roche AG, 4070 Basel (CH); Roche Diagnostics GmbH, 68305 Mannheim (DE)
(72) Erfinder: List, Hans, 64754 Hesseneck-Kailbach (DE); Leichner, Wilhelm, 68307 Mannheim (DE); Zimmer, Volker, 67229 Laumersheim (DE); Greuter, Matthias, 8952 Schlieren (CH)
(74) Vertreter: Pfiz, Thomas

(56) Entgegenhaltungen:
- EP-A- 1 360 935
- US-A1- 2006 247 555
- US-A1- 2007 038 150

## Beschreibung

Die Erfindung betrifft eine Testbandvorrichtung zum Untersuchen einer Körperflüssigkeit, insbesondere für Blutzuckertests, mit einem auf einer Spule aufwickelbaren oder aufgewickelten Trägerband, einer Vielzahl von auf dem Trägerband angeordneten Stechelementen, die mit einer in ein Körperteil einsteckbaren Spitze und einer beim Einstich gewonnene Körperflüssigkeit aufnehmenden Sammelstruktur versehen sind, und mit auf dem Trägerband aufgebrachten Testfeldern, die einem jeweiligen Stechelement zugeordnet und mit Körperflüssigkeit beaufschlagbar sind, wobei die Stechelemente jeweils über ein Koppelteil begrenzt beweglich an dem Trägerband angebracht sind.

Eine Testbandvorrichtung dieser Art ist aus der EP 1 360 935 A1 bekannt, wobei jedoch die Testfelder nicht unmittelbar auf dem Trägerband aufgebracht sind, sondern in Form von elektrochemischen sensoren an einem integrierten Stech- und Testelement angeordnet sind.

Die WO2005/107596 beschreibt ein mehrlagiges Testband, bei welchem ein Lanzetten tragender Bandstreifen sandwichartig mit einem mit Testfeldern versehenen Bandstreifen verbunden ist. Dabei stehen die Testfelder in fester Lagebeziehung zu einer Kapillarrille der Lanzetten, welche die Körperflüssigkeit über einen Kapillartransport zuführt. Die Ausrichtung der Lanzetten tangential nach vorne in Bezug auf die Bandlaufrichtung bleibt auch bei einer Bandumlenkung erhalten, so dass die Lanzettenspitzen eine relativ große Kreisbahn beschreiben, bis sie die Richtungsänderung des Bandes vollständig nachvollzogen haben. Durch die Sandwich-Anordnung wird das Gesamtgebilde auch relativ groß. Daneben sind noch Ausführungen beschrieben, bei denen die Testfelder alternierend zu den Lanzetten im Längsabstand auf dem Band angeordnet sind, um durch den Benutzer nach dem Einstich aktiv mit einem Blutstropfen beaufschlagt zu werden.

Ausgehend hiervon liegt der Erfindung die Aufgabe zugrunde, die im Stand der Technik bekannten Erzeugnisse weiter zu verbessern und so zu gestalten, dass bei kompakter Anordnung eine vereinfachte Handhabung ohne aufwändige Aktuatorik möglich ist.

Zur Lösung dieser Aufgabe wird die im unabhängigen Patentanspruch angegebene Merkmalskombination vorgeschlagen. Vorteilhafte Ausgestaltungen und Weiterbildungen der Erfindung ergeben sich aus den abhängigen Ansprüchen.

Die Erfindung geht von dem Gedanken aus, einen längeren Fließtransport der Probe von der Sammelstruktur auf das Testfeld zu vermeiden und stattdessen das Stechelement aktiv in eine fluidische Kontaktstellung zu bewegen. Dementsprechend wird erfindungsgemäß vorgeschlagen, dass ein benutztes Stechelement durch eine von dem Stechelement selbst ausgeführte Transferbewegung aus einer von einem Testfeld entfernten Gebrauchsstellung in eine Kontaktstellung in Kontakt bzw. fluidische Verbindung mit einem Testfeld bringbar ist, so dass Körperflüssigkeit aus der Sammelstruktur auf das Testfeld ummittelbar übertritt. Auf diese Weise kann das Stechelement sehr klein gehalten werden und auf eine Stechspitze mit einem Sammelvolumen reduziert werden. Der Zeitpunkt des Probentransfers lässt sich durch apparative Maßnahmen genau bestimmen. Eine zusätzliche Benutzerhandlung für den Probenübertrag ist dabei nicht erforderlich. Zugleich wird durch die feste Verbindung über das Koppelteil mit dem Band eine lagerichtige Magazinierung und einfache Entsorgung der Stechelemente gewährleistet. Zudem wird die Sterilisierung durch den Abstand zwischen Stechelement und empfindlicher Nachweischemie auf dem Testfeld im Ausgangszustand erheblich vereinfacht.

Vorteilhafterweise ist die Orientierung der Stechelemente relativ zu dem Band durch die Transferbewegung umkehrbar, wobei die Stechelemente zumindest in der Kontaktstellung in Längsrichtung des Trägerbands ausgerichtet sein können und durch eine Schwenkbewegung, insbesondere Umklappbewegung mit einem zugeordneten Testfeld in Anlage bringbar sind. Denkbar ist es jedoch auch, dass die Stechelemente mit ihrer Spitze quer zur Bandlängsrichtung weisen.

Eine weitere vorteilhafte Ausführung sieht vor, dass die Stechelemente über das Koppelteil während der Transferbewegung fest mit dem Band verbunden bleiben, so dass ein aufwändiges Loslösen nicht erforderlich ist.

Besonders bevorzugt sind die Stechelemente in einem von der Spitze beabstandeten proximalen Bereich über ein Gelenk oder Scharnier als Koppelteil an dem Trägerband angelenkt. Dies lässt sich auf einfache Weise dadurch realisieren, dass die Stechelemente über ein Biegegelenk, insbesondere ein Filmgelenk mit dem Trägerband schwenkbar verbunden sind. Eine weitere Verbesserung wird dadurch erreicht, dass die Stechelemente durch eine Folie oder Folientasche vorzugsweise steril auf dem Trägerband abgedeckt sind, wobei die Folie oder Folientasche mit dem Trägerband und einem proximalen Abschnitt des jeweiligen Stechelements fest verbunden ist.

Vorteilhafterweise besitzen die Testfelder eine Reagenzschicht, die für einen Nachweis eines Analyten in der Körperflüssigkeit ausgebildet ist.

Eine weitere besonders vorteilhafte Ausführung sieht vor, dass die Transferbewegung eines Stechelements durch Vorspulen des Trägerbands auslösbar ist, so dass kein aufwändiger Transfermechanismus erforderlich ist. Dies lässt sich auf einfache Weise dadurch verwirklichen, dass das Trägerband seitlich an einem Rückhalteteil vorbeigeführt ist, und dass das Rückhalteteil ein Anlaufhindernis für ein während des Bandtransports anlaufendes Stechelements vorzugsweise zum Umklappen des Stechelements bildet. Günstig ist es hierbei auch, wenn das Rückhalteteil im Bereich einer Umlenkstelle für das Trägerband angeordnet ist, wobei sich die Spitze der Stechelemente an der Umlenkstelle von dem Trägerband abheben lässt.

Um eine definierte Stechbewegung in Verbindung mit dem mitbewegten Band zu ermöglichen, ist es von Vorteil, wenn die Stechelemente an einer Umlenkstelle für das Trägerband durch einen Halter oder eine Klammer zur Ausführung einer Stechbewegung klemmend gehalten sind. Eine weitere Verbesserung lässt sich dadurch erreichen, dass die Umlenkstelle auf einem bei der Stechbewegung mitbewegten Schlitten angeordnet ist.

Um den Flüssigkeitstransfer zuverlässig zu gewährleisten, ist es günstig, wenn das Trägerband an einem insbesondere durch eine Blattfeder gebildeten Niederhalter vorbeigeführt ist, wobei der Niederhalter ein durchlaufendes Stechelement gegen ein Testfeld andrückt.

Für eine große Anzahl von regelmäßig durchzuführenden Selbsttests ist es für den Benutzer vorteilhaft, wenn das Trägerband als Einmalartikel vorzugsweise in Form einer Bandkassette auswechselbar ist. Gegenstand der Erfindung ist daher auch ein Testband als Disposable zum Einsetzen in eine erfindungsgemäße Testbandvorrichtung.

Im Folgenden wird die Erfindung anhand der in der Zeichnung schematisch dargestellten Ausführungsbeispiele näher erläutert. Es zeigen
- Fig. 1: eine Testbandvorrichtung in einer schaubildlichen Darstellung;
- Fig. 2: einen Testbandabschnitt mit einem auf ein Testfeld umklappbaren Stechelement in einer perspektivischen Darstellung;
- Fig. 3: bis 6 verschiedene Funktionsstellungen eines Stechelements auf dem Testband in einer Ausschnittsvergrößerung gemäß Fig. 1.

Die in der Zeichnung dargestellte Testbandvorrichtung 10 ermöglicht die Bereitstellung einer Vielzahl von disposiblen Stechelementen 12 und Testelementen 14 in Form eines Testbandes 16 für Blutglucosebestimmungen oder andere Untersuchungen an Körperflüssigkeitsproben, wobei durch eine Relativbewegung eines Stechelements zu dem Testband die Körperflüssigkeit auf ein Testfeld übertragen wird.

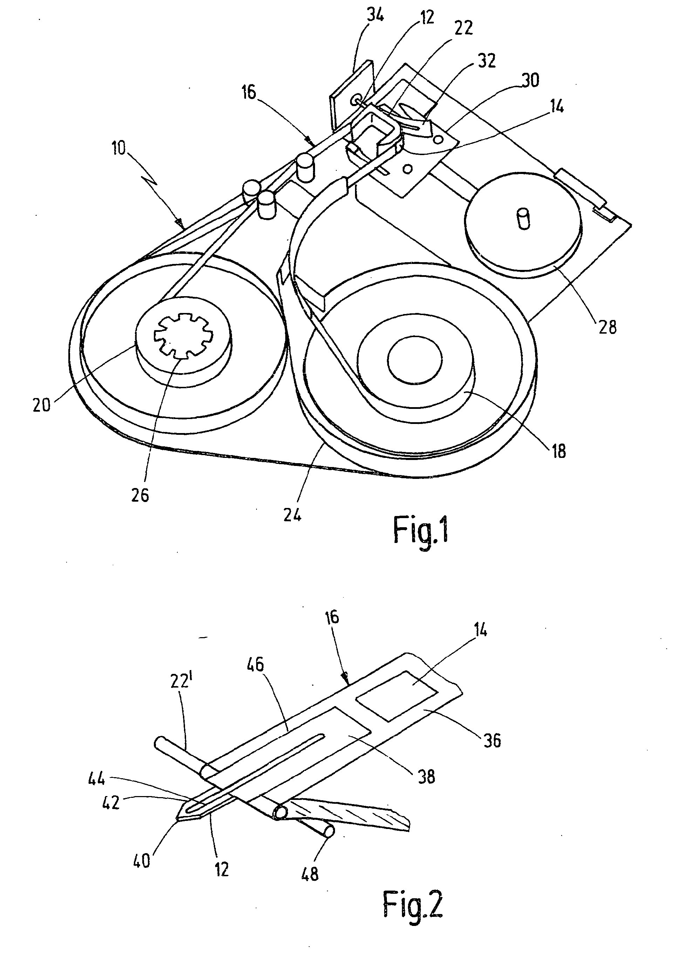

Wie in Fig. 1 veranschaulicht, ist das unverbrauchte Testband 16 von einer Vorratsspule 18 abwickelbar, während sich benutztes Bandmaterial auf einer Aufwickelspule 20 entsorgen lässt. Denkbar ist es auch, dass zumindest die Vorratsspule durch eine Kammer mit gefaltetem Band ersetzt wird. Der in Gebrauch befindliche Testbandabschnitt wird über eine Umlenkstelle 22 geführt und dabei für Messzwecke freigelegt. Bevorzugt sind die Spulen 18, 20 in Form einer Bandkassette 24 als Verbrauchsmittel vorgesehen. Auf diese Weise wird ein Bandmagazin bereitgestellt, welches eine Vielzahl von Tests erlaubt, bevor ein Verbrauchsmittelwechsel erforderlich ist.

Um Blutglucosemessungen als Selbsttest für einen Benutzer zu vereinfachen, ist die Vorrichtung 10 durch ein nur schaubildlich gezeigtes kompaktes Handgerät für einen weitgehend automatischen Messablauf konzipiert. Hierfür ist neben einem an der Aufwickelspule 20 angreifenden Bandantrieb 26 auch ein Stechantrieb 28 vorgesehen. Der Stechantrieb 28 ist für eine Linearbewegung mit einem Schlitten 30 verbunden, welcher die Umlenkstelle 22 sowie einen Greifer 32 für das Stechelement 12 trägt. Ein in eine aktive Position gebrachtes Stechelement 12 lässt sich hiermit durch einen Durchbruch einer Fingerauflage 34 in die Haut eines aufgelegten Fingers bzw. Körperteils einstechen, um ein geringes Blutvolumen für Analysezwecke zu entnehmen.

Gemäß Fig. 2 weist das Testband 14 ein flexibles Trägerband 36 auf, an welchem die Stechelemente 12 jeweils über ein Koppelteil 38 schwenkbeweglich angebracht sind, um nach der Probenaufnahme über eine Transferbewegung aus der gezeigten Gebrauchsstellung in eine Kontaktstellung in fluidische Verbindung mit einem zugeordneten Testfeld 14 gebracht zu werden.

Die auf dem Trägerband 36 magazinierten Stechelemente 12 besitzen an ihrem distalen Ende eine scharfe Spitze 40 zum Einstechen in die Haut. Der an die Spitze anschließende langgestreckte Schaft 42 ist mit einem rillen- oder schlitzförmigen Kapillarkanal 44 zum Sammeln der beim Hauteinstich gewonnenen Körperflüssigkeit, d.h. Blut und/oder Gewebeflüssigkeit versehen.

Der Stechschaft 42 ist in der Ausgangsstellung in Bandlaufrichtung des Trägerbandes ausgerichtet. In dieser Ausrichtung werden die Stechelemente 12 jeweils durch eine aus einem aufgeklebten Folienstreifen gebildete Folientasche 46 fixiert, die für eine Sterilabdeckung sorgt und zugleich im proximalen Bereich des Stechelements das Koppelelement bildet. Zu diesem Zweck ist die Folientasche 46 im hinteren Bereich fest mit dem Schaft 42 und dem Trägerband 36 verbunden. Die biegsame Folientasche 46 wirkt auf diese Weise als Filmgelenk, das sich um eine in Bandquerrichtung verlaufende Biegelinie knicken lässt, um ein Umklappen des Stechelements 12 gegen das Testfeld 14 zu erlauben.

Das Umklappen des Stechelements 12 dient als Transferbewegung, um einen Flüssigkeitstransfer auf ein auf dem Trägerband 36 nachfolgendes und im Schwenkbereich liegendes zugeordnetes Testfeld 14 zu ermöglichen. Die Steuerung dieser Bewegung allein aufgrund des Bandvorlaufs ist anhand Fig. 2 einfach verständlich. Zunächst wird an der Umlenkrolle 22' das Trägerband 36 so spitzwinklig umgelenkt, dass die Spitze 40 eines über die Umlenkrolle laufenden Stechelements 12 die Folientasche 46 durchbricht und für einen Hauteinstich freigelegt wird. Beim anschließenden Weitertransport läuft der freigelegte distale Abschnitt des Stechelements 12 gegen einen Rückhaltestift 48, welcher das Umklappen um das Koppelteil 38 der Folientasche 46 bewirkt. In der umgeklappten Kontaktstellung weist die Spitze 40 entgegen der Bandlaufrichtung, während der Kapillarkanal 44 in fluidischem Kontakt mit dem Testfeld 14 steht, um Körperflüssigkeit zu übertragen.

Für den Nachweis eines Analyten in der Körperflüssigkeit, beispielsweise Blutglucose, sind die Testfelder 14 mit einer geeigneten Reagenzschicht versehen, welche bei der Flüssigkeitsaufnahme unter Farbänderung reagiert. Diese Farbänderung lässt sich durch einen nicht gezeigten optischen Detektor erfassen und nach Messwertverarbeitung als Messergebnis für den Benutzer anzeigen.

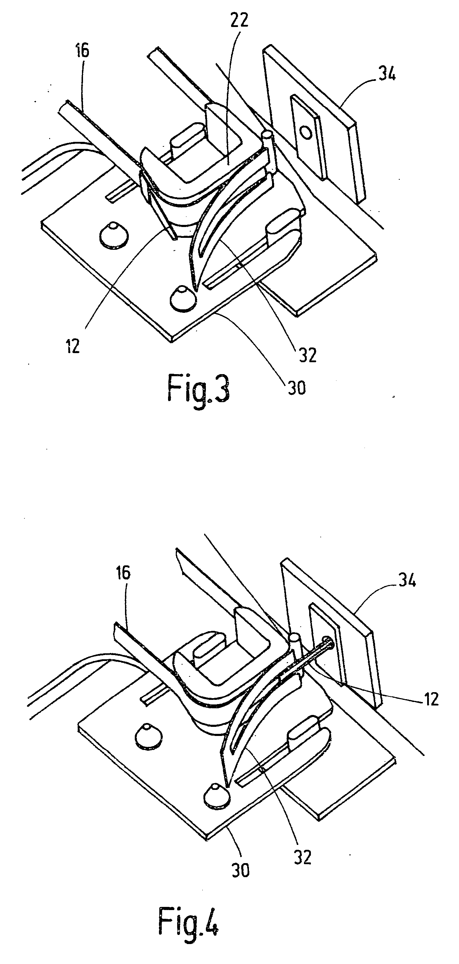

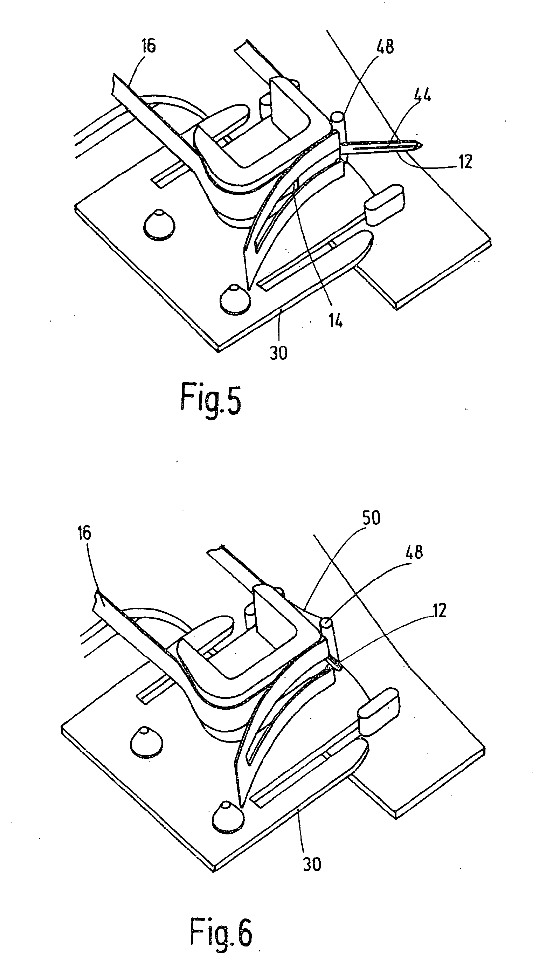

Der beispielhafte Verfahrensablauf, der nicht Teil der Erfindung ist, ist in den Fig. 3 bis 6 für das Ausführungsbeispiel nach Fig. 1 in einigen Funktionsstellungen veranschaulicht. Fig. 3 zeigt eine Fig. 2 entsprechende Situation, in der durch die Bandumlenkung an der Umlenkstelle 22 das Stechelement 12 freigelegt wird. Nach einem geeigneten Bandvorlauf wird das Stechelement 12 durch den Greifer 32 klemmend gehalten, um unter Vorschub des Schlittens 30 eine Stechbewegung durch die Fingerauflage 34 hindurch auszuführen (Fig. 4). Dabei wird der umgelenkte Bandabschnitt zusammen mit dem Stechelement 12 in Einstichrichtung bewegt. Das Stechelement 12 kann somit über das Koppelteil 38 fest mit dem Trägerband verbunden bleiben. Anschließend kann durch weiteren Bandtransport gemäß Fig. 5 die Umklappbewegung des Stechelements 14 ausgelöst werden, wobei der seitlich an dem Band angeordnete Rückhaltestift 48 als Anlaufhindernis wirkt. Schließlich wird entsprechend Fig. 6 die Kontaktstellung erreicht, in welcher die gesammelte Körperflüssigkeit von dem Stechelement 12 auf das zugeordnete Testfeld 14 übertragen wird. Dieser Kontakt kann durch einen gegen das durchlaufende Band andrückenden Niederhalter, beispielsweise in Form einer Blattfeder 50 noch verstärkt werden.

Für den gezielten Flüssigkeitstransfer ist somit weder eine gesonderte Antriebseinheit noch eine Benutzerhandlung erforderlich. Grundsätzlich ist es auch möglich, die Körperflüssigkeit über Sammelelemente aufzunehmen, die nicht für einen Einstich konzipiert sind, jedoch ein Aufnahmevolumen für eine Flüssigkeitsaufnahme besitzen.

## Patentansprüche

1. Testbandvorrichtung zum Untersuchen einer Körperflüssigkeit, insbesondere für Blutzuckertests, mit einem auf einer Spule (18,20) aufwickelbaren oder aufgewickelten Trägerband (36), einer Vielzahl von auf dem Trägerband (36) angeordneten Stechelementen (12), die mit einer in ein Körperteil einstechbaren Spitze (40) und einer beim Einstich gewonnene Körperflüssigkeit aufnehmenden Sammelstruktur (44) versehen sind, und mit auf dem Trägerband (36) aufgebrachten Testfeldern (14), die einem jeweiligen Stechelement (12) zugeordnet und mit Körperflüssigkeit beaufschlagbar sind, wobei die Stechelemente (12) jeweils über ein Koppelteil (38) beweglich an dem Trägerband (36) angebracht sind, **dadurch gekennzeichnet, dass** ein benutztes Stechelement (12) durch eine von dem Stechelement ausgeführte Transferbewegung aus einer von einem Testfeld (14) entfernten Gebrauchsstellung in eine Kontaktstellung in fluidische Verbindung mit einem Testfeld (14) bringbar ist, so dass Körperflüssigkeit aus der Sammelstruktur (44) auf das Testfeld (14) unmittelbar übertritt.

2. Testbandvorrichtung nach Anspruch 1, **dadurch gekennzeichnet, dass** die Orientierung der Stechelemente (12) relativ zu dem Trägerband (36) durch die Transferbewegung umkehrbar ist.

3. Testbandvorrichtung nach Anspruch 1 oder 2, **dadurch gekennzeichnet, dass** die langgestreckten Stechelemente (12) zumindest in der Kontaktstellung in Längsrichtung des Trägerbands (36) ausgerichtet sind.

4. Testbandvorrichtung nach einem der Ansprüche 1 bis 3, **dadurch gekennzeichnet, dass** die Stechelemente (12) durch eine Schwenkbewegung, insbesondere Umklappbewegung mit einem zugeordneten Testfeld (14) in Anlage bringbar sind.

5. Testbandvorrichtung nach einem der Ansprüche 1 bis 4, **dadurch gekennzeichnet, dass** die Stechelemente (12) über das Koppelteil (38) während der Transferbewegung mit dem Band verbunden bleiben.

6. Testbandvorrichtung nach einem der Ansprüche 1 bis 5, **dadurch gekennzeichnet, dass** die Stechelemente (12) in einem von der Spitze (40) beabstandeten proximalen Bereich über ein Gelenk oder Scharnier als Koppelteil (38) an dem Trägerband (36) angelenkt sind.

7. Testbandvorrichtung nach einem der Ansprüche 1 bis 6, **dadurch gekennzeichnet, dass** die Stechelemente (12) über ein Biegegelenk, insbesondere ein Filmgelenk mit dem Trägerband (36) schwenkbar verbunden sind.

8. Testbandvorrichtung nach einem der Ansprüche 1 bis 7, **dadurch gekennzeichnet, dass** die Stechelemente (12) durch eine Folie oder Folientasche (46) vorzugsweise steril auf dem Trägerband (36) abgedeckt sind, wobei die Folie oder Folientasche mit dem Trägerband (36) und einem proximalen Abschnitt des jeweiligen Stechelements (12) fest verbunden ist.

9. Testbandvorrichtung nach einem der Ansprüche 1 bis 8, **dadurch gekennzeichnet, dass** die Testfelder (14) eine Reagenzschicht aufweisen, die für einen Nachweis eines Analyten in der Körperflüssigkeit ausgebildet ist.

10. Testbandvorrichtung nach einem der Ansprüche 1 bis 9, **dadurch gekennzeichnet, dass** die Transferbewegung eines Stechelements (12) durch Vorspulen des Trägerbands (36) auslösbar ist.

11. Testbandvorrichtung nach einem der Ansprüche 1 bis 10, **dadurch gekennzeichnet, dass** das Trägerband (36) seitlich an einem Rückhalteteil (48) vorbeigeführt ist, und dass das Rückhalteteil (48) ein Anlaufhindernis für ein während des Bandtransports anlaufendes Stechelements (12) vorzugsweise zum Umklappen des Stechelements bildet.

12. Testbandvorrichtung nach einem der Ansprüche 1 bis 11, **dadurch gekennzeichnet, dass** das Rückhalteteil (48) im Bereich einer Umlenkstelle (22) für das Trägerband (36) angeordnet ist, wobei sich die Spitze (40) der Stechelemente (12) an der Umlenkstelle (22) von dem Trägerband (36) abhebt.

13. Testbandvorrichtung nach einem der Ansprüche 1 bis 12, **dadurch gekennzeichnet, dass** die Stechelemente (12) an einer Umlenkstelle (22) für das Trägerband (36) durch einen Greifer (32) zur Ausführung einer Stechbewegung gehalten sind.

14. Testbandvorrichtung nach einem der Ansprüche 1 bis 13, **dadurch gekennzeichnet, dass** die Umlenkstelle (22) auf einem bei der Stechbewegung mitbewegten Schlitten (30) angeordnet ist.

15. Testbandvorrichtung nach einem der Ansprüche 1 bis 14, **dadurch gekennzeichnet, dass** das Trägerband (36) an einem insbesondere durch eine Blattfeder gebildeten Niederhalter (50) vorbeigeführt ist, wobei der Niederhalter (50) ein durchlaufendes Stechelement (12) gegen ein Testfeld (14) andrückt.

16. Testbandvorrichtung nach einem der Ansprüche 1 bis 15, **dadurch gekennzeichnet, dass** das Trägerband (36) als Einmalartikel vorzugsweise in Form einer Bandkassette (24) auswechselbar ist.

## Claims

1. Test tape device for analyzing a body fluid especially for blood sugar tests having a carrier tape (36) that can be wound onto or is wound onto a spool (18, 20), a plurality of lancing elements (12) arranged on the carrier tape (36) which are provided with a tip (40) that can puncture a body part and a collecting structure (44) that takes up the body fluid obtained by the puncture, and test fields (14) mounted on the carrier tape (36) each being associated with a lancing element (12) and can have body fluid applied thereto, wherein the lancing elements (12) are each movably attached to the carrier tape (36) by a coupling member (38), **characterized in that** a used lancing element (12) can be brought into fluidic connection with a test field (14) by a transfer movement executed by the lancing element from a usage position distant from a test field (14) into a contact position, so that body fluid is transferred directly from the collecting structure (44) onto the test field (14).

2. Test tape device according to claim 1, **characterized in that** the orientation of the lancing elements (12) relative to the carrier tape (36) can be reversed by the transfer movement.

3. Test tape device according to claim 1 or 2, **characterized in that** the elongate lancing elements (12) are oriented in the longitudinal direction of the carrier tape (36) at least in the contact position.

4. Test tape device according to one of the claims 1 or 3, **characterized in that** the lancing elements (12) can be contacted with an associated test field (14) by a pivoting movement and in particular a folding movement.

5. Test tape device according to one of the claims 1 to 4, **characterized in that** the lancing elements (12) remain permanently connected to the tape by means of the coupling member (38) during the transfer movement.

6. Test tape device according to one of the claims 1 to 5, **characterized in that** the lancing elements (12) are hinged on the carrier tape (36) in a proximal region that is spaced apart from the tip (40) by means of a joint or hinge as a coupling member (38).

7. Test tape device according to one of the claims 1 to 6, **characterized in that** the lancing elements (12) are pivotally connected to the carrier tape (36) by means of a bending joint and in particular a film joint.

8. Test tape device according to one of the claims 1 to 7, **characterized in that** the lancing elements (12) are covered in a preferably sterile manner on the carrier tape (36) by a foil or foil bag (46) where the foil or foil bag is permanently connected to the carrier tape (36) and to a proximal section of the respective lancing element (12).

9. Test tape device according to one of the claims 1 to 8, **characterized in that** the test fields (14) have a reagent layer which is designed to detect an analyte in the body fluid.

10. Test tape device according to one of the claims 1 to 9, **characterized in that** the transfer movement of a lancing element (12) can be triggered by advancing the carrier tape (36).

11. Test tape device according to one of the claims 1 to 10, **characterized in that** the carrier tape (36) is guided laterally past a retaining member (48) and that the retaining member (48) forms a stop obstacle for a lancing element (12) which starts up during the tape transport preferably for folding down the lancing element.

12. Test tape device according to one of the claims 1 to 11, **characterized in that** the retaining member (48) is located in the area of a deflection point (22) for the carrier tape (36) and the tip (40) of the lancing elements (12) can be lifted from the carrier tape (36) at the deflection point (22).

13. Test tape device according to one of the claims 1 to 12, **characterized in that** the lancing elements (12) are held at a deflection point (22) for the carrier tape (36) by a gripper (32) in order to execute a lancing movement.

14. Test tape device according to one of the claims 1 to 13, **characterized in that** the deflection point (22) is arranged on a carriage (30) that is also moved during the lancing movement.

15. Test tape device according to one of the claims 1 to 14, **characterized in that** the carrier tape (36) is guided past a holding-down device (50) which is formed in particular by a leaf spring, wherein the holding-down device (50) presses a lancing element (12) which passes through against a test field (14).

16. Test tape device according to one of the claims 1 to 15, **characterized in that** the carrier tape (36) can be replaced as a single-use article preferably in the form of a tape cassette (24).

## Revendications

1. Dispositif de bande de test destiné à l'analyse d'un liquide corporel, en particulier pour des tests de glycémie, comprenant une bande support (36) enroulable ou enroulée sur une bobine (18, 20), une pluralité d'éléments de piquage (12) disposés sur la bande support (36), qui sont dotés d'une pointe (40) pouvant être piquée dans une partie du corps et d'une structure collectrice (44) recevant du liquide corporel obtenu lors du piquage, et des zones de test (14) appliquées sur la bande support (36), qui sont attribuées à un élément de piquage (12) respectif et peuvent être alimentées avec du liquide corporel, les éléments de piquage (12) étant placés respectivement au moyen d'une partie d'accouplement (38) de façon mobile sur la bande support (36), **caractérisé en ce qu'**un élément de piquage (12) utilisé peut être amené par un mouvement de transfert exécuté par l'élément de piquage à partir d'une position d'utilisation éloignée d'une zone de test (14) dans une position de contact en liaison fluidique avec une zone de test (14), de sorte que du liquide corporel passe directement de la structure collectrice (44) sur la zone de test (14).

2. Dispositif de bande de test selon la revendication 1, **caractérisé en ce que** l'orientation des éléments de piquage (12) par rapport à la bande support (36) peut être inversée par le mouvement de transfert.

3. Dispositif de bande de test selon la revendication 1 ou 2, **caractérisé en ce que** les éléments de piquage (12) étirés en longueur sont orientés au moins dans la position de contact dans la direction longitudinale de la bande support (36).

4. Dispositif de bande de test selon l'une quelconque des revendications 1 à 3, **caractérisé en ce que** les éléments de piquage (12) peuvent être amenés par un mouvement de basculement, en particulier un mouvement de rabattement, en appui avec une zone de test (14) attribuée.

5. Dispositif de bande de test selon l'une quelconque des revendications 1 à 4, **caractérisé en ce que** les éléments de piquage (12) restent reliés par la partie d'accouplement (38) à la bande pendant le mouvement de transfert.

6. Dispositif de bande de test selon l'une quelconque des revendications 1 à 5, **caractérisé en ce que** les éléments de piquage (12) sont articulés dans une zone proximale espacée de la pointe (40) au moyen d'une articulation ou d'une charnière comme partie d'accouplement (38) sur la bande support (36).

7. Dispositif de bande de test selon l'une quelconque des revendications 1 à 6, **caractérisé en ce que** les éléments de piquage (12) sont reliés de façon basculante au moyen d'une articulation de flexion, en particulier une articulation en film, à la bande support (36).

8. Dispositif de bande de test selon l'une quelconque des revendications 1 à 7, **caractérisé en ce que** les éléments de piquage (12) sont recouverts par un film ou une poche à film (46) de préférence de façon stérile sur la bande support (36), le film ou la poche à film étant relié fixement à la bande support (36) et une partie proximale de l'élément de piquage (12) respectif.

9. Dispositif de bande de test selon l'une quelconque des revendications 1 à 8, **caractérisé en ce que** les zones de test (14) présentent une couche de réactif qui est conçue pour apporter la preuve d'un analyte dans le liquide corporel.

10. Dispositif de bande de test selon l'une quelconque des revendications 1 à 9, **caractérisé en ce que** le mouvement de transfert d'un élément de piquage (12) peut être déclenché par le pré-bobinage de la bande support (36).

11. Dispositif de bande de test selon l'une quelconque des revendications 1 à 10, **caractérisé en ce que** la bande support (36) est guidée sur le côté en passant devant une partie de retenue (48), et **en ce que** la partie de retenue (48) forme un obstacle de démarrage pour un élément de piquage (12) démarrant pendant le transport de bande de préférence pour le rabattement de l'élément de piquage.

12. Dispositif de bande de test selon l'une quelconque des revendications 1 à 11, **caractérisé en ce que** la partie de retenue (48) reste dans la zone d'un endroit de renvoi (22) pour la bande support (36), la pointe (40) des éléments de piquage (12) se soulevant de la bande support (36) à l'endroit de renvoi (22).

13. Dispositif de bande de test selon l'une quelconque des revendications 1 à 12, **caractérisé en ce que** les éléments de piquage (12) sont maintenus en un endroit de renvoi (22) pour la bande support (36) par une griffe (32) pour l'exécution d'un mouvement de piquage.

14. Dispositif de bande de test selon l'une quelconque des revendications 1 à 13, **caractérisé en ce que** l'endroit de renvoi (22) est disposé sur un coulisseau (30) déplacé également lors du moment de piquage.

15. Dispositif de bande de test selon l'une quelconque des revendications 1 à 14, **caractérisé en ce que** la bande support (36) est guidée en passant devant un serre-flan (50) formé en particulier par un ressort à lame, le serre-flan (50) appuyant un élément de piquage (12) passant contre une zone de test (14).

16. Dispositif de bande de test selon l'une quelconque des revendications 1 à 15, **caractérisé en ce que** la bande support (36) peut être remplacée comme article à usage libre de préférence sous la forme d'une cassette de bande (24).
